# EUROPEAN PATENT APPLICATION

(11) **EP 1 522 225 A1**
(43) Date of publication of application: **13.04.2005**
(21) Application number: 04009418.7
(22) Date of filing: 21.04.2004
(51) Int. Cl.: A23L 1/00, A23L 1/22, A61K 9/00

(54) **Bilayer edible sheet**

(30) Priority: 10.10.2003 US 681903; 07.04.2004 US 819164
(71) Applicant: ORB Co., Ltd., Osaka-shi Osaka (JP)
(72) Inventor: Nakamura, Hiroshi, Yodogawa-ku Osaka-shi Osaka (JP)
(74) Representative: Schnappauf, Georg Dr.

(57) **Abstract**

There is provided an edible sheet obtainable by steps of: producing a base sheet layer by mixing ingredients such as starch with water and drying a mixture with a hot air to produce a base sheet layer; mixing functional ingredients such as flavoring agents with a particular organic solvent; applying a mixture of functional ingredients on the base sheet; and drying the mixture with a air to form a functional ingredient layer on the base sheet layer. The edible sheet of the present invention can solve problems such as occurrence of hardening, hygroscopicity, fragility, discoloration and the like due to coexistence of the functional ingredients such as flavoring agents, acidulants, pharmaceutically active agents and the like with other ingredients.

## Description

### FIELD OF THE INVENTION

The present invention relates to a bilayer edible sheet and a process for producing the same. More particularly, the present invention relates to an edible sheet in a food or medicinal field, in which functional ingredients can immediately melt upon ingestion to provide a desired flavorsome, delicious feeling or a pharmaceutical activity to a subject, and which does not show physical fragility, hardening and discoloration and shows suppressed hygroscopicity during a prolonged period storage after production as well as a process for producing the same.

### BACKGROUND OF THE INVENTION

Hitherto, all edible sheets utilized in a food or medicinal field have been a monolayer film which contains all ingredients in a single layer. Consequently, when various functions are imparted to the edible sheet, ingredients essential for constitution of the edible sheet and functional ingredients such as acidulants, flavoring agents, medicines and the like have been mixed together to prepare the monolayer edible sheet.

However, when the ingredients essential for constitution of the edible sheet and the functional ingredients are contained together in a single layer, there arise problems that physical properties of the sheet are modified to cause fragility, hardening, discoloration and hygroscopicity of the sheet.

For example, in order to impart fruit-like flavor to the edible sheet, the edible sheet is conventionally prepared by adding flavoring agents such as spices, concentrated juice or powdered juice to a mixture of sheet-constituting ingredients. But, there arise problems that when a certain juice is mixed with the sheet-constituting ingredients, hardening of the edible sheet is caused due to an interaction between the juice and the sheet-constituting ingredients, or when an acidulant is mixed with the sheet-constituting ingredients, fragility and discoloration of the edible sheet are caused due to a lowered pH.

Also in a pharmaceutical edible sheet, there arise similar problems when active ingredients to be contained chemically modify the sheet-constituting ingredients.

Accordingly, in order to avoid above problems, there are problems that a kind or an amount of the ingredients to be contained in the edible sheet must be limited, or a storage duration or condition of the edible sheet must be limited within a particular range. In addition, in a drying procedure of the sheet, there are problems that a drying temperature, a humidity or temperature of a drying air, or the like must be limited within a particular range.

### SUMMARY OF THE INVENTION

In view of such situations, the inventor studied intensively and, as the result, found that above problems can be solved by producing a bilayer edible sheet in which the sheet-constituting ingredients and the functional ingredients which affect the sheet-constituting ingredient to deteriorate physical properties of the edible sheet are contained in separated layers, which resulted in completion of the present invention.

Specifically, the inventor found that the edible sheet can be produced by applying on a base sheet layer a mixture of functional ingredients, with an organic solvent which does not substantially dissolve the base sheet layer, resulting in solving the problems as described above. In the edible sheet, the functional ingredients are separated from the sheet-constituting ingredients, and the edible sheet can melt immediately upon ingestion to exert its functions.

That is, in a first aspect, the present invention provides a bilayer edible sheet obtainable by steps of:
(1) mixing at least one ingredient selected from the group consisting of a starch, a modified starch, a protein, a protein hydrolysate and a gum with water, uniformly applying the mixture on a supporting sheet, and drying the mixture with a hot air to produce a base sheet layer on the supporting sheet;
(2) mixing a surface active agent and at least one functional ingredient selected from the group consisting of a flavoring agent, an acidulant and a pharmaceutically active agent with an organic solvent which does not substantially dissolve the base sheet layer at 30°C to prepare a mixture of functional ingredients;
(3) applying the mixture of functional ingredients prepared in the step (2) on the base sheet layer produced in the step (1), and air-drying the mixture to form a functional ingredient layer on the base sheet layer; and
(4) removing the base sheet layer from the supporting sheet.

In addition, in another aspect, the present invention provides a process for producing a bilayer edible sheet comprising steps of:
(1) mixing at least one ingredient selected from the group consisting of a starch, a modified starch, a protein, a protein hydrolysate and a gum with water, uniformly applying the mixture on a supporting sheet, and drying the mixture with a hot air to produce a base sheet layer on the supporting sheet;
(2) mixing a surface active agent and at least one functional ingredient selected from the group consisting of a flavoring agent, an acidulant and a pharmaceutically active agent with an organic solvent which does not substantially dissolve the base sheet layer at 30°C to prepare a mixture of functional ingredients;
(3) applying the mixture of functional ingredients prepared in the step (2) on the base sheet layer produced in the step (1), and air-drying the mixture to form a functional ingredient layer on the base sheet layer; and
(4) removing the base sheet layer from the supporting sheet.

According to the present invention, an edible sheet and a process for producing it in a food or medicinal field can be provided, which can immediately melt upon ingestion to provide a desired flavorsome, delicious feeling or a pharmaceutical activity to a subject. In addition, the edible. sheet of the present invention does not cause fragility, hardening, discoloration and hygroscopicity over a prolonged period storage after production.

Ingredients which constitute a base sheet layer of the edible sheet of the present invention include those contained in a conventional food or medicinal edible sheet. For example, these include a starch, a modified starch, a protein, a protein hydrolysate, a gum, and the like. These ingredients may be contained alone or in combination. For example, a combination of a modified protein and a protein is preferable. In addition, two or more kinds of the same ingredients may be used. For example, two or more kinds of starches may be used.

Specifically referring to individual ingredients, the starch includes, for example, rice starch, corn starch, tapioca starch, potato starch, green gram starch, and the like. Such starches may be contained alone or in combination in an amount of 0-90% by weight based on a total weight of ingredients contained in a dried base sheet layer.

The modified starches includes, for example, Amycol No.6-L^{R} (Nippon Starch Chemical Co., Ltd.), Foodtex^{R} (Matsutani Chemical Industry Co., Ltd.), and the like. Such modified starches may be contained alone or in combination in an amount of 0-90% by weight based on a total weight of ingredients contained in a dried base sheet layer.

The protein or the protein hydrolysate includes, for example, gelatin, gelatin hydrolysate, casein, casein hydrolysate, and the like. Such proteins or protein hydrolysates may be contained alone or in combination in an amount of 0-90% by weight based on a total weight of ingredients contained in a dried base sheet layer.

The gum includes, for example, carrageenan, gum arabic, propylene glycol alginate, sodium alginate, purified konjac extracts (PROPOL^{R} (Shimizu Chemical Corporation)), and the like. Such gums may be contained alone or in combination in an amount of 0-10% by weight based on a total weight of ingredients contained in a dried base sheet layer. A concentration of a gum in an aqueous mixture of base sheet ingredients is 0-6% by weight, preferably 0.4-5% by weight, and more preferably 0.4-4% by weight.

A total concentration of a starch, a modified starch, a protein, a protein hydrolysate and/or a gum in an aqueous mixture of base sheet ingredients is 20-50% by weight, preferably 25-45% by weight, and more preferably 32-39% by weight.

Moreover, in addition to the above ingredients, a sweetening agent, a surface active agent, a plasticizer, a coloring agent, a sheet-reinforcing agent, or the like may be optionally contained in the base sheet layer of the edible sheet of the present invention.

The sweetening agent includes, for example, aspartame (Palsweet^{R} (Ajinomoto Co., Ltd.)), acesulfame potassium (Sunett^{R} (Nutrinova Japan Co., Ltd.)), licorice extracts, and the like. Such sweetening agents may be contained alone or in combination in an amount of 0-30% by weight based on a total weight of ingredients contained in a dried base sheet layer. A concentration of a sweetening agent in an aqueous mixture of base sheet ingredients is 0-15% by weight, preferably 0-10% by weight, and more preferably 0-5% by weight.

The surface active agent includes, for example, glycerin fatty acid ester, sucrose fatty acid ester, propylene glycol fatty acid ester, and the like. Such surface active agents may be contained alone or in combination in an amount of 0-10% by weight based on a total weight of ingredients contained in a dried base sheet layer. A concentration of a surface active agent in an aqueous mixture of base sheet ingredients is 0-6% by weight, preferably 0.4-5% by weight, and more preferably 0.4-4% by weight.

The plasticizer includes, for example, glycerol, propylene glycol, glycerin fatty acid ester, medium chain triglyceride (Panasate^{R} (NOF Corporation) or the like), and the like. Such plasticizers may be contained alone or in combination in an amount of 0-10% by weight based on a total weight of ingredients contained in a dried base sheet layer. A concentration of a plasticizer in an aqueous mixture of base sheet ingredients is 0-5% by weight, preferably 0-4% by weight, and more preferably 0-2% by weight.

The coloring agent includes, for example, FD&C Yellow No. 5 (Food yellow No. 4 in Japan (tartrazine)), FD&C Red No. 3 (Food red No. 3 in Japan (erythrocin)), FD&C Blue No. 1 (Food blue No. 1 in Japan (brilliant blue)), caramel, and the like. Such coloring agents may be contained alone or in combination in an amount of 0-5% by weight based on a total weight of ingredients contained in a dried base sheet layer. A concentration of a coloring agent in an aqueous mixture of base sheet ingredients is 0-3% by weight, preferably 0-2% by weight, and more preferably 0-1% by weight.

The sheet-reinforcing agent includes, for example, fats and oils, fatty acid esters, wax, and the like. Such sheet-reinforcing agents may be contained alone or in combination in an amount of 0-10% by weight based on a total weight of ingredients contained in a dried base sheet layer. A concentration of a sheet-reinforcing agent in an aqueous mixture of base sheet ingredients is 0-6% by weight, preferably 0.35-5% by weight, and more preferably 0.4-3% by weight.

A base sheet layer of the edible sheet can be produced by dissolving or dispersing above ingredients, if necessary with heating, into water to prepare a mixture, uniformly applying the mixture on a supporting sheet such as a synthetic resin sheet and the like using a coater, a bar coater, or the like, and drying with a hot air according to a conventional procedure for producing edible sheets. Alternatively, depending on the ingredients constituting the base sheet layer, the base sheet layer can be produced by merely applying the mixture on a fluorine- or silicon-processed endless belt conveyer, drying with a hot air, peeling off, winding up in a roll form, and the like, without using the supporting sheet. A thickness of a dried base sheet layer of the edible sheet is 10-200µ m, preferably 10-50µ m, and more preferably 20-50µ m.

When a thickness of the base sheet is below 10µ m, a mechanical strength of the sheet is lowered, thereby, peeling of the sheet from the supporting sheet in a final step becomes difficult. On the other hand, when a thickness exceeds 200µ m, problems such as a partial peeling or crack may arise upon drying due to a different coefficient of thermal expansion between the supporting sheet and the base sheet. Accordingly, such ranges are not preferable.

Moreover, functional ingredients, which are contained in the edible sheet of the present invention for imparting various functions thereto, include ingredients which are normally used in a food or medicinal field, and which may adversely effect on the edible sheet as described above upon mixing with ingredients to be contained in the base sheet layer. Such functional ingredients include, for example, a flavoring agent, an acidulant, a pharmaceutically active agent and the like. Specifically, the flavoring agent includes, for example, fruit flavor, cinnamon flavor, coffee flavor, and the like. Such flavoring agents may be contained alone or in combination in an amount of 0-20% by weight based on a total weight of ingredients contained in a dried edible sheet. A concentration of a flavoring agent in a mixture of functional ingredients in an organic solvent is 0-15% by weight, preferably 2-10% by weight, and more preferably 3-7% by weight.

Moreover, the acidulant includes, for example, citric acid, tartaric acid, malic acid, fumaric acid, salts thereof, and the like. Such acidulants may be contained alone or in combination in an amount of 0-20% by weight based on a total weight of ingredients contained in a dried edible sheet. A concentration of an acidulant in a mixture of functional ingredients in an organic solvent is 0-15% by weight, preferably 0-10% by weight, and more preferably 0-7% by weight.

Moreover, the pharmaceutically active agent includes, for example, antipyretic and analgesic agents such as acetaminophen and ethenzamide; central nervous system stimulants such as caffeine and anhydrous caffeine; sympathomimetic agents such as dl-enphedrine hydrochloride and phenylpropanolamine hydrochloride; antihistamines such as clemastine fumarate and chlorpheniramine maleate; antitussives such as noscapine hydrochloride and tipepidine citrate; antigenous drugs such as scopolamine hydrobromide; anti-inflammatory enzymes such as lysozyme; expectorants such as potassium guiaiacolsulfonate; crude drugs such as licorice root powder; vitamins such as pyridoxine hydrochloride, vitamin B1, vitamin B2, vitamin B6, vitamin B12, vitamin C, vitamin E and calcium pautothenate; amino acids such as arginine, lysine, phenylalanine and glutamine. Such pharmaceutically active agents may be contained alone or in combination in an amount of 0-80% by weight based on a total weight of ingredients contained in a dried edible sheet. A concentration of a pharmaceutically active agent in a mixture of functional ingredients in an organic solvent is 0-80% by weight, preferably 0-70% by weight, and more preferably 0-60% by weight. Furthermore, various ingredients other than the ingredients as described above which do not adversely effect on the edible sheet may be contained in the edible sheet of the present invention.

Moreover, a surface active agent may be contained in the edible sheet of the present invention in order to dissolve or disperse above functional ingredients in an organic solvent as described below. The surface active agent includes, for example, glycerin fatty acid ester, sucrose fatty acid ester, and the like. Such surface active agents may be contained alone or in combination in an amount of 0-3% by weight based on a total weight of ingredients contained in a dried edible sheet. A concentration of a surface active agent in a mixture of functional ingredients in an organic solvent is 0-3% by weight, preferably 0-2% by weight, and more preferably 0-1.5% by weight.

An organic solvent, which is used for preparing a mixture of functional ingredients, may be any one which does not substantially dissolve the base sheet layer, preferably at 20-40°C and more preferably at 30°C, and which has a low boiling point and may be normally used for producing the edible sheet in a food or medicinal field. The organic solvent includes, for example, acetone, ethyl alcohol, methylene chloride, esters such as amyl acetate, and the like, but preferably is selected from the group consisting of acetone, ethyl alcohol and esters. Such organic solvents may be contained alone or in combination so long as they satisfy the requirements as described above. Although an amount of an organic solvent is not particularly limited so long as it can homogeneously dissolve or disperse the functional ingredients to be used, preferably is suitable for applying the mixture of functional ingredients on the base sheet layer and drying it in a relatively short time. An amount of an organic solvent is, for example, half to three times (weight/weight), preferably half to two times (weight/weight), and more preferably one to one and half time (weight/weight) based on a total amount of functional ingredients except for the organic solvent.

A mixture of functional ingredients can be prepared by dissolving or dispersing the functional ingredients as described above, if necessary with heating, into an organic solvent.

As used herein, the phrase "organic solvent which does not substantially dissolve ..." refers to an organic solvent which does not dissolve the base sheet layer by a visual detection, even when it is contacted with the base sheet layer at room temperature for a time necessary for volatizing the solvent.

Moreover, a drying aid may be optionally contained in the mixture of functional ingredients of the edible sheet of the present invention in order to stabilize the mixture of functional ingredients applied on the base sheet layer or to promote drying of the mixture. The drying aid is not particularly limited so long as it does not dissolve in the mixture of functional ingredients and it can suppress flowing of the mixture of functional ingredients applied on the base sheet layer. In addition, the drying aid should have an average diameter so that drying of the mixture of functional ingredients can be promoted due to an increase in a surface area of the mixture of functional ingredients. The drying aid includes, for example, spherical or formless powder such as corn starch; rice starch, fumaric acid, and the like. An average diameter of such a powder is preferably 5-50µ m, and more preferably 5-20µ m. When the average diameter of powder is below 5µ m, it becomes difficult to suppress flowing of the mixture of functional ingredients or promote drying of the mixture. On the other hand, when it exceeds 50µ m, the edible sheet looks granular and, consequently, an appearance of the edible sheet is deteriorated, being not preferable. Such drying aids may be contained alone or in combination in an amount of 0-40% by weight based on a total weight of ingredients contained in the dried edible sheet. A concentration of a drying aid in a mixture of functional ingredients in an organic solvent is 0-40% by weight, preferably 3-30% by weight, and more preferably 5-15% by weight.

Moreover, an adhesive may be optionally contained in the mixture of the functional ingredients of the edible sheet of the present invention in order to fix the drying aid on the base sheet layer or to prevent hygroscopicity of the edible sheet. The adhesive may be any one which can be used in an edible sheet in a food or medicinal field so long as it can stably fix the drying aid to the base sheet layer and can prevent hygroscopicity of the edible sheet under a usual storage or use condition. The adhesive includes, for example, shellac, cellulose derivative, glycerin fatty acid ester, and the like. Such adhesives may be contained alone or in combination in an amount of 0-10% by weight based on a total amount of ingredients contained in the dried edible sheet. A concentration of an adhesive in the mixture of functional ingredients in an organic solvent is 0-10% by weight, preferably 2-7% by weight, and more preferably 3-5% by weight. In addition, the adhesive may be directly added to the mixture of functional ingredients, but it may be added to the mixture after it is prepared into a solution by dissolving in another organic solvent in order to enhance solubility thereof in an organic solvent. For example, when an ester is used as an organic solvent, a solution of shellac in ethyl alcohol is prepared in advance, and the solution is added to the ester into which the functional ingredients have been mixed while stirring, to prepare a mixture of functional ingredients.

Next, the mixture of functional ingredients prepared is applied to the base sheet layer produced in advance. Application of the mixture of functional ingredients can be conducted using any conventional procedures used in a food or medicinal field. For example, application using a coater or a bar coater, application using offset printing, gravure printing or screen printing, spraying with a sprayer, application using an electrostatic applicator, and application by a casting method may be used. The mixture of functional ingredients may be uniformly applied on the base sheet layer such that the base sheet layer is covered with the functional ingredient layer.

Alternatively, the mixture of functional ingredients may be streakily applied on the base sheet layer such that the dried functional ingredient layer forms a particular figure such as a strip, a variety of designs, and the like. Accordingly, when the mixture of functional ingredients is streakily applied on the base sheet layer, there are portions on the base sheet layer where the functional ingredient layer is present and where it is not present. A ratio of an area of the base sheet layer where the functional ingredient layer is present relative to a sum of an area of the base sheet layer where the functional ingredient layer is present and an area of the base sheet layer where the functional ingredient layer is not present may be 30-100%, preferably 35-100%, and more preferably 40-100%.

When a plurality of edible sheets are stored in a stacked manner, the edible sheet sometimes adhere to neighbor sheets, and it becomes difficult to take one sheet from stacked sheets. In this embodiment, even when a plurality of bilayer edible sheets are stored in a stacked manner, one sheet can be easily taken from stacked edible sheets, since air is present between the stacked sheets, an area of the sheet attaching to other sheets is decreased and, thereby, a sliding ability of the sheet is enhanced. Moreover, in this embodiment, the bilayer edible sheet can immediately melt in an oral cavity upon ingestion due to an increased surface area and, thereby, an effect of the functional ingredients is immediately exerted. Also, in the pharmaceutical edible sheet, a pharmacological activity of the functional ingredients is immediately exerted. Moreover, a variety of figures as the functional ingredient layer can be drawn on the base sheet layer to enhance aesthetics of the edible sheet. Moreover, the mixture can be dried to produce the bilayer edible sheet in a shorter time than in the case where the mixture is uniformly applied due to the increased surface area. In addition, the edible sheet of this embodiment appears thick compared with one prepared by uniformly applying the same amount of the mixture on the base sheet layer.

Moreover, when the mixture of functional ingredients is streakily applied such that stripes as the functional ingredient layer are formed on the base sheet layer, cracking of the functional ingredient layer along the stripes can be prevented and, thereby, the functional ingredient layer is hardly peeled off from the base sheet layer.

Furthermore, the bilayer edible sheet which is produced by uniformly applying the mixture of functional ingredients on the base sheet layer may sometimes curl during a storage due to a difference in a coefficient of expansion between the functional ingredient layer and the base sheet layer, but such curling can be highly suppressed when the mixture of functional ingredients is applied on the base sheet layer in a stripe manner.

Next, the mixture of functional ingredients applied on the base sheet layer is dried. Drying is preferably conducted at a low to ambient temperature in the light of suppression of denaturation or volatilization of the functional ingredients contained in the mixture, but drying may be generally conducted under various conditions depending on an organic solvent to be used. For example, drying may be conducted at 20-50°C for acetone, at 35-80°C for ethyl alcohol, at 20-40°C for methylene chloride, or at 20-40°C for the esters. Since the mixture of functional ingredients can be dried at a relatively low temperature and in a very short time, volatilization of a flavoring agent or the like contained as a functional ingredient can be suppressed, thereby, the edible sheet can be produced without deteriorating its flavor.

The mixture of functional ingredients of the edible sheet may be applied on the base sheet layer such that a thickness of a functional ingredient layer, which is formed after drying the mixture of functional ingredients, becomes 5-100µ m, preferably 10-40µ m, and more preferably 10-20µ m.

Finally, when the base sheet layer is produced using a supporting sheet as described above, the supporting sheet is peeled off from the base sheet layer after drying the mixture of functional ingredients to obtain a bilayer edible sheet of the present invention. Of course, when the base sheet layer is produced without the supporting sheet, it is not required to peel off it.

As stated above, the bilayer edible sheet of the present invention which is constituted from the base sheet layer and the functional ingredient layer can be produced by drying the mixture of functional ingredients applied on the base sheet layer. Since the ingredients contained in the base sheet layer and those contained in the functional ingredient layer are separated, the edible sheet which solves the problems as stated above can be produced.

The edible sheet of the present invention thus produced has a total thickness, a sum of a thickness of the base sheet layer and that of the functional ingredient layer, of 15-300µ m, preferably 40-80µ m, and more preferably 30-60µ m. Thereafter, the edible sheet of the present invention can be cut into a suitable size, packaged or the like to obtain a final product.

### EXAMPLES

Next, the present invention will be illustrated more specifically with referring to working examples, but the present invention is not limited thereto.

### Example 1

### Production of Base Sheet Layer

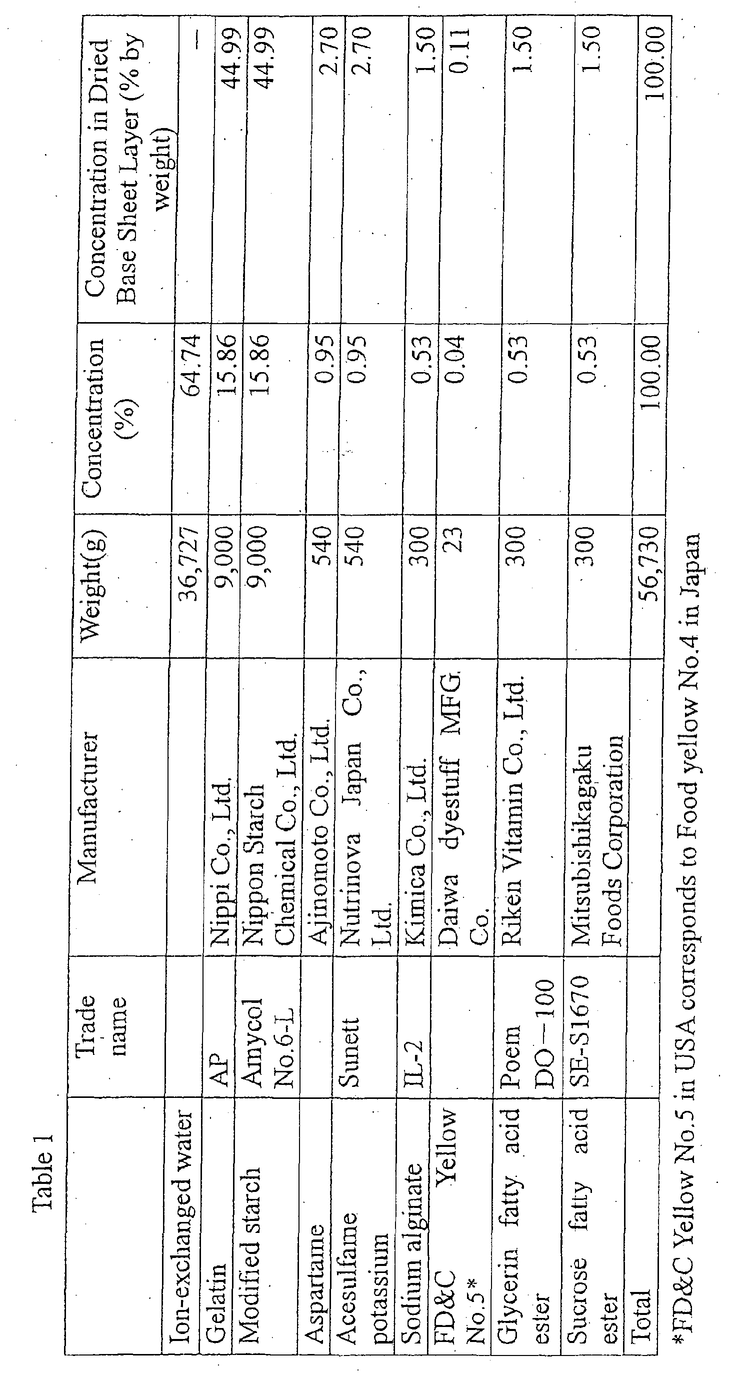

Into a 100 L stainless steel internal mixer equipped with an agitator, ion-exchanged water was placed, and then gelatin in Table 1 was added with stirring. Mixing was continued with heating so that a temperature of the mixture was maintained at 85°C using a jacket. After approximately 30 min, the mixture became a homogenous semi-translucent yellow liquid. Then, other ingredients in Table 1 were added and dissolved into the mixture with stirring, and the mixture was degassed. The mixture was applied to a polyethylene terephthalate film having a 60µ m thickness (SUNTOX Co., Ltd.) using a coater (Shinko Co., Ltd.) which had been set so that a thickness of a dried sheet becomes approximately 50µ m.

The mixture applied on the film was dried by passing through a hot-air dryer (Shinko Co., Ltd.) regulated at 75°C over approximately 5 min to obtain a base sheet layer of the edible sheet having a 50µ m thickness.

### Example 2

**Table 2**

| | Ingredient | Weight (g) | % |
|---|---|---|---|
| 1 | Corn starch | 2,500 | 27 |
| 2 | Anhydrous citric acid | 2,500 | 27 |
| 3 | Ethyl alcohol | 2,500 | 27 |
| 4 | 20% Shellac solution in ethyl alcohol | 150 | 2 |
| 5 | Lemon oil | 1,500 | 16 |
| 6 | Glycerin fatty acid ester | 150 | 2 |
| | Total | 9,300 | 100 |

Ingredients 1-6 in Table 2 were mixed, degassed at a lower temperature of 20°C or lower *in vacuo* to prepare a muddy mixture of functional ingredients. The mixture was well stirred, applied to the base sheet layer having a 50µ m thickness which had been produced in Example 1 at a mixture thickness of 40µ m using a coater (Shinko Co., Ltd.), and the mixture was dried with a hot air at 40°C. A thickness of the dried functional ingredient layer was 17µ m, and a lemon-flavored edible sheet having a total thickness, a sum of thicknesses of two layers, of 67µ m was produced.

### Example 3

**Table 3**

| | Ingredient | Weight (g) | % |
|---|---|---|---|
| 1 | Corn starch | 2,500 | 28 |
| 2 | Sodium carboxymethylcellulose | 50 | 1 |
| 3 | Anhydrous citric acid | 2,000 | 23 |
| 4 | Ethyl alcohol | 2,500 | 28 |
| 5 | 20% Shellac solution in ethyl alcohol | 100 | 1 |
| 6 | Orange oil | 1,500 | 17 |
| 7 | Sucrose fatty acid ester | 150 | 2 |
| | Total | 8,800 | 100 |

Ingredients 1-7 in Table 3 were mixed, degassed at a lower temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 55µ m, using a bar coater (Shinko Co., Ltd.), and the mixture was dried with a hot air at 40°C. A thickness of the dried functional ingredient layer was 20µ m, and an orange-flavored edible sheet having a total thickness, a sum of thicknesses of two layers, of 75µ m was produced.

### Example 4

**Table 4**

| | Ingredient | Weight (g) | % |
|---|---|---|---|
| 1 | Green gram starch | 2,500 | 28 |
| 2 | Amyl acetate | 50 | 1 |
| 3 | Anhydrous citric acid | 2,000 | 23 |
| 4 | Methylene chloride | 2,500 | 28 |
| 5 | 20% Shellac solution in ethyl alcohol | 100 | 1 |
| 6 | Orange oil | 1,500 | 17 |
| 7 | Sucrose fatty acid ester | 150 | 2 |
| | Total | 8,800 | 100 |

Ingredients 1-7 in Table 4 were mixed, degassed at a lower temperature of 20°C or lower *in vacuo* to prepare a muddy mixture of functional ingredients. The mixture was well stirred, applied at a mixture thickness of 25µ m to a base sheet layer which had been produced according to the same manner as that of Example 1 except that a thickness thereof was set at 30µ m, using a gravure printing machine (Shinko Co., Ltd.), and the mixture was dried with a hot air at 30°C. A thickness of the dried functional ingredient layer was 17µ m, and an orange-flavored edible sheet having a total thickness, a sum of thicknesses of two layers, of 47µ m was produced.

### Example 5

**Table 5**

| | Ingredient | Weight (g) | % |
|---|---|---|---|
| 1 | Corn starch | 2,500 | 27 |
| 2 | Fumaric acid | 1,000 | 11 |
| 3 | Anhydrous citric acid | 1,500 | 16 |
| 4 | Ethyl alcohol | 2,500 | 27 |
| 5 | Sodium carboxymethylcellulose | 50 | 1 |
| 6 | 20% Shellac solution in ethyl alcohol | 100 | 1 |
| 7 | Lemon oil | 1,500 | 16 |
| 8 | Sucrose fatty acid ester | 150 | 2 |
| | Total | 9,300 | 100 |

Ingredients 1-8 in Table 5 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 70µ m, using a silk screen printing machine (Shinko Co., Ltd.), and the mixture was dried with a hot air at 40°C. A thickness of the dried functional ingredient layer was 19µ m, and a lemon-flavored edible sheet having a total thickness, a sum of thicknesses of two layers, of 89µ m was produced.

### Example 6

**Table 6**

| | Ingredient | Weight (g) | % |
|---|---|---|---|
| 1 | Fumaric acid | 2,500 | 27 |
| 2 | Rice starch | 1,000 | 11 |
| 3 | Anhydrous citric acid | 1,500 | 16 |
| 4 | Ethyl alcohol | 2,500 | 27 |
| 5 | 20% Shellac solution in ethyl alcohol | 100 | 1 |
| 6 | Strawberry oil , | 1,500 | 16 |
| 7 | Glycerin fatty acid ester | 150 | 2 |
| | Total | 9,250 | 100 |

Ingredients 1-6 in Table 6 were mixed, degassed at a low temperature of 20 °C or lower *in vacuo* to prepare a muddy mixture of functional ingredients. The mixture was well stirred, applied at a mixture thickness of 30µ m to a base sheet layer which had been produced according to the same manner as that of Example 1 except that a thickness thereof was set at 70µ m, using a coater (Shinko Co., Ltd.), and the mixture was dried with a hot air at 40°C. A thickness of the dried functional ingredient layer was 11 µ m, and a strawberry-flavored edible sheet having a total thickness, a sum of thicknesses of two layers, of 81µ m was produced.

### Example 7

**Table 7**

| | Ingredient | Weight (g) | % |
|---|---|---|---|
| 1 | Rice starch | 1,950 | 19 |
| 2 | Anhydrous citric acid | 2,600 | 26 |
| 3 | Ethyl alcohol | 3,250 | 32 |
| 4 | 20% Shellac solution in ethyl alcohol | 195 | 2 |
| 5 | Peach essence | 1,950 | 19 |
| 6 | Glycerin fatty acid ester | 195 | 2 |
| | Total | 10,140 | 100 |

Ingredients 1-6 in Table 7 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 50µ m, using a bar coater (Shinko Co., Ltd.), and the mixture was dried with a hot air at 40°C. A thickness of the dried functional ingredient layer was 19µ m, and a peach-flavored edible sheet having a total thickness, a sum of thicknesses of two layers, of 69µ m was produced.

### Example 8

**Table 8**

| | Ingredient | Weight (g) | % |
|---|---|---|---|
| 1 | Corn starch | 1,950 | 19 |
| 2 | Coffee oil | 2,600 | 26 |
| 3 | Ethyl alcohol | 3,250 | 32 |
| 4 | 20% Shellac solution in ethyl alcohol | 195 | 2 |
| 5 | Coffee extract powder | 1,950 | 19 |
| 6 | Glycerin fatty acid ester | 195 | 2 |
| | Total | 10,140 | 100 |

Ingredients 1-6 in Table 8 were mixed, degassed at a low temperature of 20°C or lower *in vacuo* to prepare a muddy mixture of functional ingredients. The mixture was well stirred, applied at a mixture thickness of 50µ m to a base sheet layer which had been produced according to the same manner as that of Example 1 except that a thickness thereof was set at 50µ m, using a spray (Shinko Co., Ltd.), and the mixture was dried with a hot air at 40°C. A thickness of the dried functional ingredient layer was 17 µ m, and a coffee-flavored edible sheet having a total thickness, a sum of thicknesses of two layers, of 67µ m was produced.

### Example 9

**Table 9**

| | Ingredient | Weight (g) | % |
|---|---|---|---|
| 1 | Wheat starch | 2,100 | 21 |
| 2 | Hot pepper oil | 3,500 | 34 |
| 3 | Ethyl alcohol | 3,500 | 34 |
| 4 | 20% Shellac solution in ethyl alcohol | 210 | 2 |
| 5 | Menthol | 700 | 7 |
| 6 | Glycerin fatty acid ester | 210 | 2 |
| | Total | 10,220 | 100 |

Ingredients 1-6 in Table 9 were mixed, degassed at a low temperature of 20°C or lower *in vacuo* to prepare a muddy mixture of functional ingredients. The mixture was well stirred, applied at a mixture thickness of 30µ m to a base sheet layer which had been produced according to the same manner as that of Example 1 except that a thickness thereof was set at 35µ m, using a coater (Shinko Co., Ltd.), and the mixture was dried with a hot air at 40°C. A thickness of the dried functional ingredient layer was 10 µ m, and a hot pepper-flavored edible sheet having a total thickness, a sum of thicknesses of two layers, of 45µ m was produced.

### Example 10

**Table 10**

| | Ingredient | Weight (g) | % |
|---|---|---|---|
| 1 | Rice starch | 2,400 | 21 |
| 2 | Green tea powder | 4,000 | 34 |
| 3 | Ethyl alcohol | 4,800 | 41 |
| 4 | 20% Shellac solution in ethyl alcohol | 240 | 2 |
| 5 | Glycerin fatty acid ester | 240 | 2 |
| | Total | 11,680 | 100 |

Ingredients 1-5 in Table 10 were mixed, degassed at a low temperature of 20°C or low *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 50µ m, using a coater (Shinko Co., Ltd.), and the mixture was dried with a hot air at 40°C. A thickness of the dried functional ingredient layer was 18µ m, and a green tea-flavored edible sheet having a total thickness, a sum of thicknesses of two layers, of 68µ m was produced.

### Example 11

**Table 11**

| | Ingredient | Weight (g) | % |
|---|---|---|---|
| 1 | Rice starch | 2,400 | 22 |
| 2 | Tea powder | 1,600 | 15 |
| 3 | Ethyl alcohol | 4,000 | 37 |
| 4 | 20% Shellac solution in ethyl alcohol | 240 | 2 |
| 5 | Menthol | 800 | 7 |
| 6 | Glycerin fatty acid ester | 240 | 2 |
| 7 | Tea flavor | 800 | 7 |
| 8 | Lemon oil | 400 | 4 |
| 9 | Anhydrous citric acid | 400 | 4 |
| | Total | 10,880 | 100 |

Ingredients 1-9 in Table 11 were mixed, degassed at a low temperature of 20 °C or lower *in vacuo* to prepare a muddy mixture of functional ingredients. The mixture was well stirred, applied to a base sheet layer which had been produced according to the same manner as that of Example 1 except that a thickness thereof was set at 50µ m, using a coater (Shinko Co., Ltd.), and the mixture was dried with a hot air at 40°C. A thickness of the dried functional ingredient layer was 18µ m, and a tea with lemon-flavored edible sheet having a total thickness, a sum of thicknesses of two layers, of 68µ m was produced.

### Example 12

**Table 12**

| | Ingredient | Weight (g) | % |
|---|---|---|---|
| 1 | Rice starch | 1,800 | 19 |
| 2 | Japanese apricot and perilla oil | 1,200 | 13 |
| 3 | Ethyl alcohol | 3,000 | 31 |
| 4 | 20% Shellac solution in ethyl alcohol | 180 | 2 |
| 5 | Menthol | 600 | 6 |
| 6 | Glycerin fatty acid ester | 180 | 2 |
| 7 | Japanese apricot fruit extract | 600 | 6 |
| 8 | Lemon oil | 120 | 1 |
| 9 | Anhydrous citric acid | 1,800 | 19 |
| | Total | 9,480 | 100 |

Ingredients 1-9 in Table 12 were mixed, degassed at a low temperature at 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 40µ m, using a coater (Shinko Co., Ltd.), and the mixture was dried with a hot air at 40°C. A thickness of the dried functional ingredient layer was 17 µ m, and a pickled Japanese apricot-flavored edible sheet having a total thickness, a sum of thicknesses of two layers, of 57µ m was produced.

### Example 13

**Table 13**

| | Ingredient | Weight (g) | % |
|---|---|---|---|
| 1 | Rice starch | 2,100 | 19 |
| 2 | Grape oil | 1,400 | 13 |
| 3 | Ethyl alcohol | 4,200 | 38 |
| 4 | 20% Shellac solution in ethyl alcohol | 210 | 2 |
| 5 | Sucrose fatty acid ester | 210 | 2 |
| 6 | Anhydrous citric acid | 2,800 | 26 |
| | Total | 10,920 | 100 |

Ingredients 1-6 in above Table 13 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 50µ m, using a bar coater (Shinko Co., Ltd.), and the mixture was dried with a hot air at 40°C. A thickness of the dried functional ingredient layer was 17µ m, and a grape mint-flavored edible sheet having a total thickness, a sum of thicknesses of two layers, of 67µ m was produced.

### Comparative Examples 1-13

A monolayer edible sheet as Comparative Example 1 was produced by substituting a 20% by weight of anhydrous citric acid (acidulant) for ion-exchanged water in a procedure for producing the base sheet layer as described in Example 1. Moreover, monolayer edible sheets as Comparative Examples 2-13 were produced by mixing the ingredients of the functional ingredient layer used in Examples 2-13 and the ingredients of the base sheet layer described in Example 1, respectively, according to the conventional procedures.

### Evaluation of Edible Sheet Stability

The bilayer edible sheets of Examples 2-13 and the monolayer edible sheets of Comparative Examples 2-13 were placed in separate polyethylene bags, respectively. The bags were heat-sealed, and stored under darkness at 36°C for 6 months. Thereafter, the edible sheets were recovered, and they were evaluated for discoloration by a visible observation, for hardness by a hand feeling, and for fragility by folding the sheet and determining whether it is divided. The results thereof are shown in Tables 14 and 15.

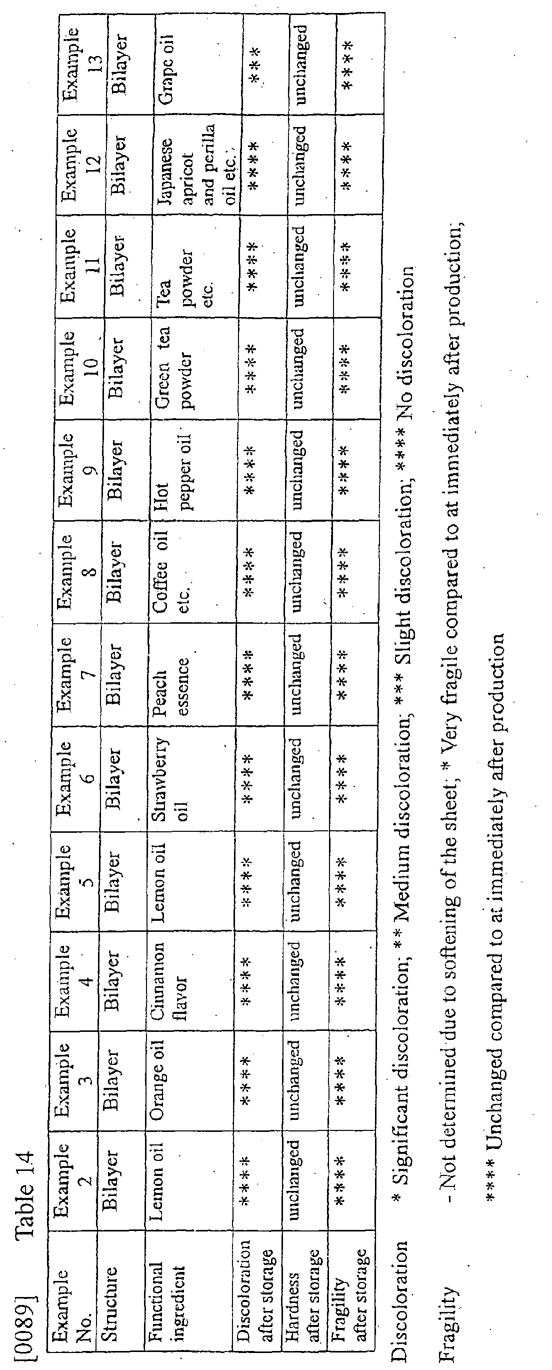

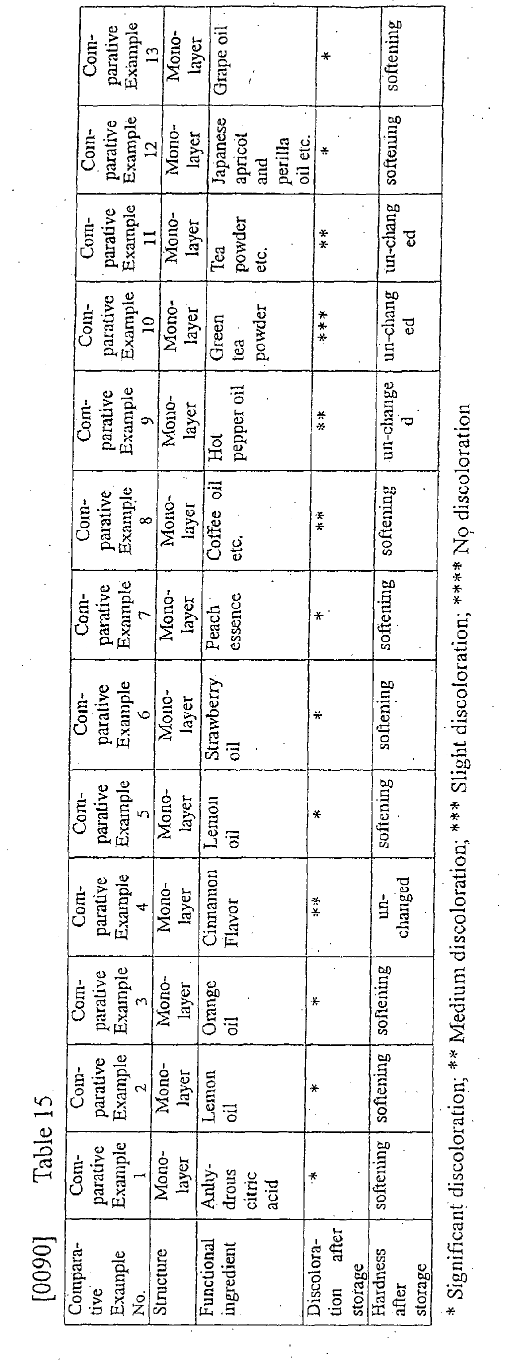

As shown in Tables 14 and 15, it was confirmed that bilayer edible sheets of Examples 2-13 caused no discoloration, hardening, fragility of the sheet even after 6 months storage. In contrast, it was confirmed that monolayer edible sheets of Comparative Examples 1-13 caused early discoloration, softening, and fragility.

### Evaluation of Stripe Type Bilayer Edible Sheet

The mixture of functional ingredients was prepared from the ingredients 1-6 in Table 8 as described above. Then, the mixture was streakily applied on the base sheet layer which had been produced according to the same manner as that of Example 1 except that a thickness thereof was set at 65µ m, using a coater (Shinko Co., Ltd.) such that the mixture forms 10 stripes on the base sheet layer having 2.2cm width, and the mixture was dried by running it in a hot-air dryer (National EH599, Matsushita Electronic Industry, Co., Ltd.) at a speed of 10m/min to produce a bilayer edible sheet (Stripe type bilayer edible sheet). On the other hand, the same amount of the mixture was uniformly applied on the base sheet layer to produce another bilayer edible sheet (Uniform type bilayer edible sheet). Between such two types of bilayer edible sheets, a drying speed, a thickness, and meltability in an oral cavity were measured and evaluated. The results thereof are shown in Table 16. A thickness of the bilayer edible sheet was measured using a thickness gauge (Teclock Co.).

**Table 16**

| | Uniform type bilayer edible sheet | Stripe type bilayer edible sheet |
|---|---|---|
| Drying speed (Time required for drying) | 45sec. | 28sec. |
| Thickness | 85µ m | 100µ m |
| Meltability (Time required for melt) | 40sec. | 25sec. |

As shown in Table 16, it was confirmed that the stripe type bilayer edible sheet can be dried faster than the uniform type bilayer edible sheet. Moreover, when the bilayer edible sheet was produced using the same amount of the mixture of functional ingredients, it was confirmed that the stripe type bilayer edible sheet has a thicker appearance than that of the uniform type bilayer edible sheet. Furthermore, it was confirmed that the stripe type bilayer edible sheet can melt in an oral cavity faster than the uniform type bilayer edible sheet. Therefore, it was found that the bilayer edible sheet produced by streakily applying the mixture of functional ingredients has a variety of superior properties to those of the bilayer edible sheet produced by uniformly applying the mixture of functional ingredients.

### Example 14

### Production of Base Sheet Layer

All ingredients of Table 1 were mixed according to the same manner as that of Example 1, stirred at 60 to 70°C to dissolve, and degassed. The mixture was applied to a supporting sheet (polyethylene terephthalate film having a 60 µ m thickness (SUNTOX Co., Ltd.)), and dried by passing through a hot-air dryer (Shinko Co., Ltd.) regulated at 75°C over approximately 5 min to obtain a base sheet layer of the edible sheet having a 50µ m thickness.

### Example 15

**Table 17**

| | Ingredient | Weight (g) | % |
|---|---|---|---|
| 1 | Acetaminophen | 9,000 | 23.04 |
| 2 | Ethenzamide | 4,320 | 11.06 |
| 3 | Caffeine | 2,160 | 5.53 |
| 4 | Propylene glycol | 270 | 0.69 |
| 5 | Glycerol | 270 | 0.69 |
| 6 | Ethyl alcohol | 21,600 | 55.30 |
| 7 | Methylcellulose | 180 | 0.46 |
| 8 | Glycerin fatty acid ester | 360 | 0.92 |
| 9 | Lemon oil | 900 | 2.30 |
| | Total | 39,060 | 100.00 |

Ingredients 1-9 in Table 17 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 60µ m, using a bar coater (Shinko Co., Ltd.), and the mixture was dried with a hot air at 40°C. A thickness of the dried functional ingredient layer was 42µ m, and a bilayer edible sheet having a total thickness, a sum of thicknesses of two layers, of 102µ m was produced. After peeling the film, on which the base sheet layer had produced, from the edible sheet, a lemon-flavored bilayer edible sheet having an antipyretic and analgesic effect was produced.

### Example 16

**Table 18**

| | Ingredient | Weight (g) | % |
|---|---|---|---|
| 1 | Acetaminophen | 9,000 | 22.46 |
| 2 | Lysozyme chloride | 600 | 1.50 |
| 3 | Potassium guaiacolsulfonate | 2,400 | 5.99 |
| 4 | dl-Ephedrine hydrochroride | 600 | 1.50 |
| 5 | Noscapine hydrochloride | 1,350 | 3.37 |
| 6 | Clemastine fumarate | 1,350 | 3.37 |
| 7 | Caffeine | 750 | 1.87 |
| 8 | Benfotiamin | 240 | 0.60 |
| 9 | Propylene glycol | 75 | 0.19 |
| 10 | Glycerol | 75 | 0.19 |
| 11 | Ethyl alcohol | 22,750 | 56.75 |
| 12 | Lemon oil | 500 | 1.25 |
| 13 | Methylcellulose | 250 | 0.62 |
| 14 | Glycerin fatty acid ester | 150 | 0.37 |
| | Total | 40,090 | 100.00 |

Ingredients 1-14 in Table 18 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 60µ m, using a gravure printing machine (Shinko Co., Ltd.), and the mixture was dried with a hot air at 40°C. A thickness of the dried functional ingredient layer was 38µ m, and a bilayer edible sheet having a total thickness, a sum of thicknesses of two layers, of 98µ m was produced. After peeling the film, on which the base sheet layer had produced, from the edible sheet, a lemon-flavored bilayer edible sheet having a common cold treating effect was produced.

### Example 17

**Table 19**

| | Ingredient | Weight (g) | % |
|---|---|---|---|
| 1 | Phenylpropanolamine hydrochloride | 5,940 | 15.97 |
| 2 | Chlorpheniramine maleate | 792 | 2.13 |
| 3 | Anhydrous Caffeine | 7,920 | 21.29 |
| 4 | Propylene glycol | 165 | 0.44 |
| 5 | Glycerol | 165 | 0.44 |
| 6 | Ethyl alcohol | 19,250 | 51.74 |
| 7 | Lemon oil | 1,650 | 4.44 |
| 8 | Methylcellulose | 660 | 1.77 |
| 9 | Glycerin fatty acid ester | 660 | 1.77 |
| | Total | 37,202 | 100.00 |

Ingredients 1-9 in Table 19 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 60µ m, using a silk screen printing machine (Shinko Co., Ltd.), and the mixture was dried with a hot air at 40°C. A thickness of the dried functional ingredient layer was 40µ m, and a bilayer edible sheet having a total thickness, a sum of thicknesses of two layers, of 100µ m was produced. After peeling the film, on which the base sheet layer had produced, from the edible sheet, a lemon-flavored bilayer edible sheet having a rhinitis treating effect was produced.

### Example 18

**Table 20**

| | Ingredient | Weight (g) | % |
|---|---|---|---|
| 1 | Licorice powder | 5,940 | 16.28 |
| 2 | Tipepidine citrate | 792 | 2.17 |
| 3 | Dextrin | 7,920 | 21.71 |
| 4 | Noscapine | 165 | 0.45 |
| 5 | Propylene glycol | 330 | 0.90 |
| 6 | Glycerol | 330 | 0.90 |
| 7 | Ethyl alcohol | 18,700 | 51.25 |
| 8 | Lemon oil | 1,650 | 4.52 |
| 9 | Methylcellulose | 330 | 0.90 |
| 10 | Glycerin fatty acid ester | 330 | 0.90 |
| | Total | 36,487 | 100.00 |

Ingredients 1-10 in Table 20 were mixed, degassed at a low temperature of 20 °C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 60µ m, using a bar coater (Shinko Co., Ltd.), and the mixture was dried with a hot air at 40°C. A thickness of the dried functional ingredient layer was 42µ m, and a bilayer edible sheet having a total thickness, a sum of thicknesses of two layers, of 102µ m was produced. After peeling the film, on which the base sheet layer had produced, from the edible sheet, a lemon-flavored bilayer edible sheet having an antitussive effect was produced.

### Example 19

**Table 21**

| | Ingredient | Weight (g) | % |
|---|---|---|---|
| 1 | Ascorbic acid | 13,750 | 35.26 |
| 2 | Lysozyme chloride | 495 | 1.26 |
| 3 | Tocopherol chloride | 495 | 1.26 |
| 4 | Sodium copper chlorophylin | 330 | 0.85 |
| 5 | Propylene glycol | 275 | 0.70 |
| 6 | Glycerol | 275 | 0.70 |
| 7 | Ethyl alcohol | 20,900 | 53.60 |
| 8 | Orange oil | 1,100 | 2.82 |
| 9 | Methylcellulose | 1,100 | 2.82 |
| 10 | Glycerin fatty acid ester | 275 | 0.70 |
| | Total | 38,995 | 100.00 |

Ingredients 1-10 in Table 21 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 60µ m, using a bar coater (Shinko Co., Ltd.), and the mixture was dried with a hot air at 40°C. A thickness of the dried functional ingredient layer was 40µ m, and a bilayer edible sheet having a total thickness, a sum of thicknesses of two layers, of 100µ m was produced. After peeling the film, on which the base sheet layer had produced, from the edible sheet, an orange-flavored bilayer edible sheet having a mouth sterilizing effect was produced.

### Example 20

**Table 22**

| | Ingredient | Weight (g) | % |
|---|---|---|---|
| 1 | Ethyl aminobenzoate | 7,000 | 19.35 |
| 2 | Scopolamine hydrobromide | 28 | 0.08 |
| 3 | Anhydrous caffeine | 2,800 | 7.74 |
| 4 | Scopolamine maleate | 4,200 | 11.61 |
| 5 | Pyridoxine hydrochloride | 700 | 1.93 |
| 6 | Propylene glycol. | 280 | 0.77 |
| 7 | Glycerol | 280 | 0.77 |
| 8 | Ethyl alcohol | 18,900 | . 52.24 |
| 9 | Lemon oil | 1,400 | 3.87 |
| 10 | Methylcellulose | 280 | 0.77 |
| 11 | Glycerin fatty acid ester | 280 | 0.77 |
| | Total | 36,180 | 100.00 |

Ingredients 1-11 in Table 22 were mixed, degassed at a low temperature of 20 °C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 60µ m, using a bar coater (Shinko Co., Ltd.), and the mixture was dried with a hot air at 40°C. A thickness of the dried functional ingredient layer was 44µ m, and a bilayer edible sheet having a total thickness, a sum of thicknesses of two layers, of 104µ m was produced. After peeling the film, on which the base sheet layer had produced, from the edible sheet, a lemon-flavored bilayer edible sheet having a motion sickness treating effect was produced.

### Example 21

**Table 23**

| | Ingredient | Weight (g) | % |
|---|---|---|---|
| 1 | Vitamin B1 | 8,000 | 20.19 |
| 2 | Vitamin B2 | 32 | 0.08 |
| 3 | VitaminB12 | 3,200 | 8.07 |
| 4 | Vitamin E | 4,800 | 12.11 |
| 5 | Propylene glycol | 160 | 0.40 |
| 6 | Glycerol | 160 | 0.40 |
| 7 | Ethyl alcohol | 21,200 | 53.49 |
| 8 | Orange oil | 1,600 | 4.04 |
| 9 | Methylcellulose | 320 | 0.80 |
| 10 | Glycerin fatty acid ester | 160 | 0.40 |
| | Total | 39,632 | 100.00 |

Ingredients 1-10 in Table 23 were mixed, degassed at a low temperature of 20 °C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 65µ m, using a bar coater (Shinko Co., Ltd.), and the mixture was dried with a hot air at 40°C. A thickness of the dried functional ingredient layer was 40µ m, and a bilayer edible sheet having a total thickness, a sum of thicknesses of two layers, of 105µ m was produced. After peeling the film, on which the base sheet layer had produced, from the edible sheet, an orange-flavored bilayer edible sheet having a vitamins supplying effect was produced.

### Example 22

**Table 24**

| | Ingredient | Weight (g) | % |
|---|---|---|---|
| 1 | VitaminE | 10,000 | 28.95 |
| 2 | Hepronicate | 40 | 0.12 |
| 3 | VitaminB1 | 4,000 | 11.58 |
| 4 | Propylene glycol | 200 | 0.58 |
| 5 | Glycerol | 200 | 0.58 |
| 6 | Ethyl alcohol | 16,500 | 47.77 |
| 7 | Spearmint oil | 2,000 | 5.79 |
| 8 | Methylcellulose | 400 | 1.16 |
| 9 | Lemon oil | 1,000 | 2.90 |
| 10 | Glycerin fatty acid ester | 200 | 0.58 |
| | Total | 34,540 | 100.00 |

Ingredients 1-10 in Table 24 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 70µ m, using a bar coater (Shinko Co., Ltd.), and the mixture was dried with a hot air at 40°C. A thickness of the dried functional ingredient layer was 42µ m, and a bilayer edible sheet having a total thickness, a sum of thicknesses of two layers, of 112µ m was produced. After peeling the film, on which the base sheet layer had produced, from the edible sheet, a lemon-flavored bilayer edible sheet having a blood circulation promoting effect was produced.

### Example 23

**Table 25**

| | Ingredient | Weight (g) | % |
|---|---|---|---|
| 1 | VitaminC | 7,000 | 18.52 |
| 2 | VitaminB 1 | 700 | 1.85 |
| 3 | VitaminB6 | 700 | 1.85 |
| 4 | Anhydrous caffeine | 4,200 | 11.11 |
| 5 | Nicotinic acid amide | 2,800 | 7.40 |
| 6 | Propylene glycol | 140 | 0.37 |
| 7 | Glycerol | 140 | 0.37 |
| 8 | Ethyl alcohol | 20,300 | 53.70 |
| 9 | Lemon oil | 1,400 | 3.70 |
| 10 | Methylcellulose | 280 | 0.74 |
| 11 | Glycerin fatty acid ester | 140 | 0.37 |
| | Total | 37,800 | 100.00 |

Ingredients 1-11 in Table 25 were mixed, degassed at a low temperature of 20 °C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 60µ m, using a bar coater (Shinko Co., Ltd.), and the mixture was dried with a hot air at 40°C. A thickness of the dried functional ingredient layer was 40µ m, and a bilayer edible sheet having a total thickness, a sum of thicknesses of two layers, of 100µ m was produced. After peeling the film, on which the base sheet layer had produced, from the edible sheet, a lemon-flavored bilayer edible sheet having a nutrients supplying effect was produced.

### Example 24

**Table 26**

| | Ingredient | Weight (g) | % |
|---|---|---|---|
| 1 | Ferric pyrophosphate | 14,400 | 39.01 |
| 2 | Cyanocobalamin | 9 | 0.02 |
| 3 | Folic acid | 360 | 0.98 |
| 4 | Propylene glycol | 360 | 0.98 |
| 5 | Glycerol | 360 | 0.98 |
| 6 | Ethyl alcohol | 18,900 | 51.21 |
| 7 | Lemon oil | 1,800 | 4.88 |
| 8 | Methylcellulose | 540 | 1.46 |
| 9 | Glycerin fatty acid ester | 180 | 0.49 |
| | Total | 36,909 | 100.00 |

Ingredients 1-9 in Table 26 were mixed, degassed at a low temperature of 20 °C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 55µ m, using a bar coater (Shinko Co., Ltd.), and the mixture was dried with a hot air at 40°C. A thickness of the dried functional ingredient layer was 44µ m, and a bilayer edible sheet having a total thickness, a sum of thicknesses of two layers, of 99µ m was produced. After peeling the film, on which the base sheet layer had produced, from the edible sheet, a lemon-flavored bilayer edible sheet having an anemia treating effect was produced.

### Example 25

**Table 27**

| | Ingredient | Weight (g) | % |
|---|---|---|---|
| 1 | Pyridoxine hydrochloride | 7,700 | 19.54 |
| 2 | Caffeine | 7,000 | 17.76 |
| 3 | Propylene glycol | 140 | 0.36 |
| 4 | Glycerol | 140 | 0.36 |
| 5 | Ethyl alcohol | 21,000 | 53.29 |
| 6 | Peppermint oil | 1,050 | 2.66 |
| 7 | Menthol | 1,400 | 3.55 |
| 8 | Methylcellulose | 210 | 0.53 |
| 9 | Lemon oil | 700 | 1.78 |
| 10 | Glycerin fatty acid ester | 70 | 0.18 |
| | Total | 39,410 | 100.00 |

Ingredients 1-10 in Table 27 were mixed, degassed at a low temperature of 20 °C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 60µ m, using a bar coater (Shinko Co., Ltd.), and the mixture was dried with a hot air at 40 °C. A thickness of the dried functional ingredient layer was 44µ m, and a bilayer edible sheet having a total thickness, a sum of thicknesses of two layers, of 104µ m was produced. After peeling the film, on which the base sheet layer had produced, from the edible sheet, a lemon-flavored bilayer edible sheet having a drowsiness curing effect was produced.

### Example 26

**Table 28**

| | Ingredient | Weight (g) | % |
|---|---|---|---|
| 1 | Nicotine | 40 | 0.10 |
| 2 | Cellulose | 7,500 | 18.78 |
| 3 | Menthol | 1,000 | 2.50 |
| 4 | Dextrin | 7,500 | 18.78 |
| 5 | Propylene glycol | 200 | 0.50 |
| 6 | Glycerol | 200 | 0.50 |
| 7 | Ethyl alcohol | 22,250 | 55.71 |
| 8 | Tobacco flavor | 750 | 1.88 |
| 9 | Methylcellulose | 400 | 1.00 |
| 10 | Glycerin fatty acid ester | 100 | 0.25 |
| | Total | 39,940 | 100.00 |

Ingredients 1-10 in Table 28 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 65µ m, using a bar coater (Shinko Co., Ltd.), and the mixture was dried with a hot air at 45°C. A thickness of the dried functional ingredient layer was 46µ m, and a bilayer edible sheet having a total thickness, a sum of thicknesses of two layers, of 111µ m was produced. After peeling the film, on which the base sheet layer had produced, from the edible sheet, a tobacco-flavored bilayer edible sheet having a no smoking subsidiary effect was produced.

### Example 27

**Table 29**

| | Ingredient | Weight (g) | % |
|---|---|---|---|
| 1 | Arginine | 7,140 | 17.99 |
| 2 | Lysine | 3,920 | 9.88 |
| 3 | Phenylalanine | 1,540 | 3.88 |
| 4 | Glutamine | 3,360 | 8.47 |
| 5 | Propylene glycol | 280 | 0.71 |
| 6 | Glycerol | 280 | 0.71 |
| 7 | Ethyl alcohol | 21,000 | 52.91 |
| 8 | Orange oil | 1,400 | 3.53 |
| 9 | Methylcellulose | 560 | 1.41 |
| 10 | Glycerin fatty acid ester | 210 | 0.53 |
| | Total | 39,690 | 100.00 |

Ingredients 1-10 in Table 29 were mixed, degassed at a low temperature of 20°C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 50µ m, using a bar coater (Shinko Co., Ltd.), and the mixture was dried with a hot air at 38°C. A thickness of the dried functional ingredient layer was 40µ m, and a bilayer edible sheet having a total thickness, a sum of thicknesses of two layers, of 90µ m was produced. After peeling the film, on which the base sheet layer had produced, from the edible sheet, an orange-flavored bilayer edible sheet having an amino acids supplying effect was produced.

### Example 28

**Table 30**

| | Ingredient | Weight (g) | % |
|---|---|---|---|
| 1 | Vitamin B1 | 5,240 | 14.71 |
| 2 | Calcium pantothenate | 1,600 | 4.46 |
| 3 | Vitamin B6 | 6,400 | 17.83 |
| 4 | Vitamin B12 | 272 | 0.76 |
| 5 | Oryzanol | 272 | 0.76 |
| 6 | Vitamin E | 272 | 0.76 |
| 7 | Nicotinic acid amide | 512 | 1.43 |
| 8 | Propylene glycol | 320 | 0.89 |
| 9 | Glycerol | 320 | 0.89 |
| 10 | Ethyl alcohol | 18,400 | 51.27 |
| 11 | Lemon oil | 1,600 | 4.46 |
| 12 | Methylcellulose | 320 | 0.89 |
| 13 | Glycerin fatty acid ester | 320 | 0.89 |
| | Total | 35,888 | 100.00 |

Ingredients 1-13 in Table 30 were mixed, degassed at a low temperature of 20 °C or lower *in vacuo* to prepare a muddy mixture of functional ingredients. The mixture was well stirred, applied at a mixture thickness of 60µ m to a base sheet layer which had been produced according to the same manner as that of Example 1 except that a thickness thereof was set at 55µ m, using a bar coater (Shinko Co., Ltd.), and the mixture was dried with a hot air at 45°C. A thickness of the dried functional ingredient layer was 40µ m, and a bilayer edible sheet having a total thickness, a sum of thicknesses of two layers, of 95µ m was produced. After peeling the film, on which the base sheet layer had produced, from the edible sheet, a bilayer edible sheet having a vitamins supplying effect was produced.

### Comparative Examples 14-28

A monolayer edible sheet as Comparative Example 14 was produced by substituting a 20% by weight of anhydrous citric acid (acidulant) for ion-exchanged water in a procedure for producing the base sheet layer as described in Example 1. Moreover, monolayer edible sheets as Comparative Examples 15-28 were produced by mixing the ingredients of the functional ingredient layer used in Examples 15-28 and the ingredients of the base sheet layer described in Example 14, respectively, according to the conventional procedures.

### Evaluation of Edible Sheet Stability

The bilayer edible sheets of Examples 15-28 and the monolayer edible sheets of Comparative Examples 14-28 were placed in separate polyethylene bags, respectively. The bags were heat-sealed, and stored under darkness at 36°C for 6 months. Thereafter, the edible sheets were recovered, and they were evaluated for discoloration by a visible observation, for hardness by a hand feeling, and for fragility by folding the sheet and determining whether it is divided. The results thereof are shown in Tables 31 and 32.

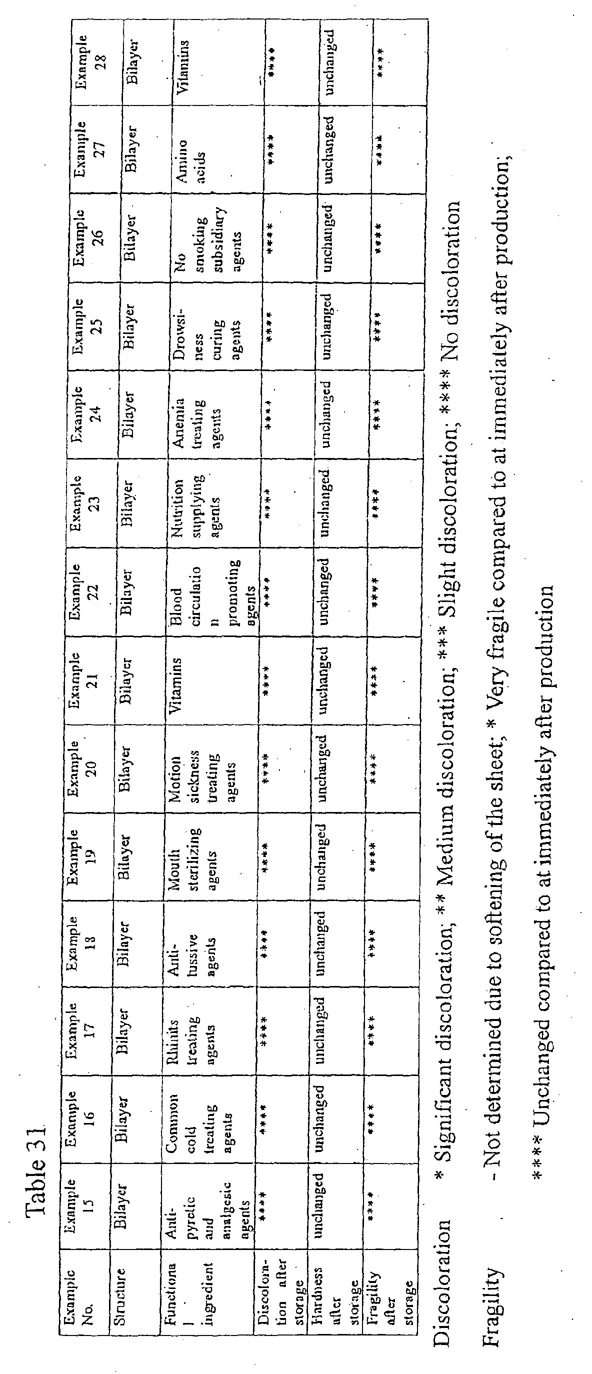

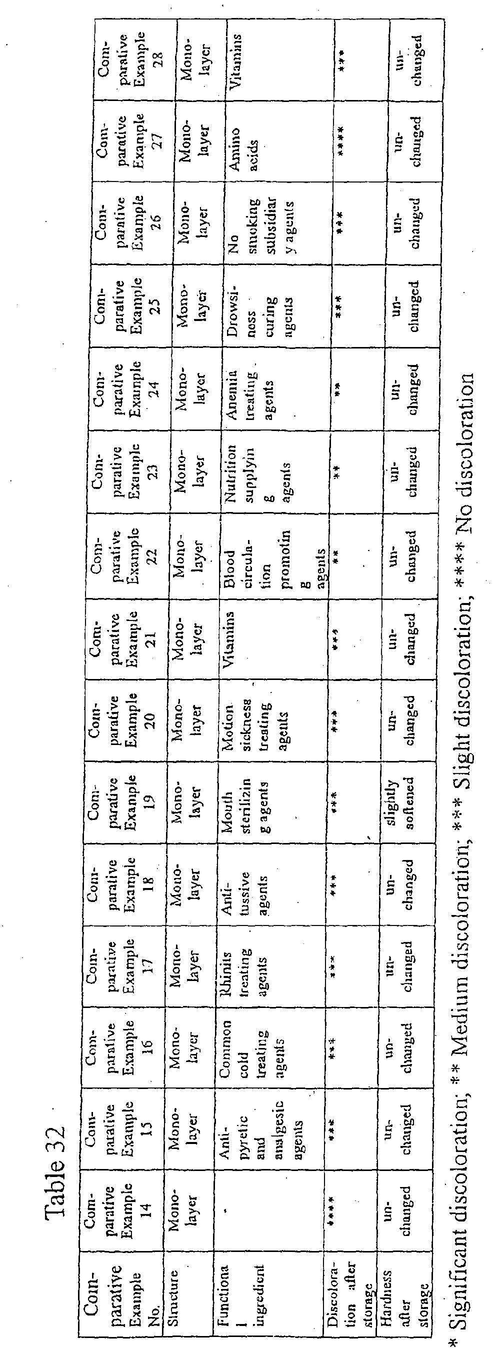

As shown in Tables 31 and 32, it was confirmed that bilayer edible sheets of Examples 15-28 caused no discoloration, hardening, fragility of the sheet even after 6 months storage. In contrast, it was confirmed that monolayer edible sheets of Comparative Examples 14-28 caused early discoloration, softening, and fragility.

### Evaluation of Stripe Type Bilayer Edible Sheet

The mixture of functional ingredients was prepared from the ingredients 1-10 in Table 23 as described above. Then, the mixture was streakily applied on the base sheet layer which had been produced according to the same manner as that of Example 1 except that a thickness thereof was set at 65µ m, using a coater (Shinko Co., Ltd.) such that the mixture forms 10 stripes on the base sheet layer having 2.2cm width, and the mixture was dried by running it in a hot-air dryer (National EH599, Matsushita Electronic Industry, Co., Ltd.) at a speed of 10m/min to produce a bilayer edible sheet (Stripe type bilayer edible sheet). On the other hand, the same amount of the mixture was uniformly applied on the base sheet layer to produce another bilayer edible sheet (Uniform type bilayer edible sheet). Between such two types of bilayer edible sheets, a drying speed, a thickness, and meltability in an oral cavity were measured and evaluated. The results thereof are shown in Table 33. A thickness of the bilayer edible sheet was measured using a thickness gauge (Teclock Co.).

**Table 33**

| | Uniform type bilayer edible sheet | Stripe type bilayer edible sheet |
|---|---|---|
| Drying speed (Time required for drying) | 45sec. | 28sec. |
| Thickness | 85µ m | 100µ m |
| Meltability (Time required for melt) | 40sec. | 25sec. |

As shown in Table 33, it was confirmed that the stripe type bilayer edible sheet can be dried faster than the uniform type bilayer edible sheet. Moreover, when the bilayer edible sheet was produced using the same amount of the mixture of functional ingredients, it was confirmed that the stripe type bilayer edible sheet has a thicker appearance than that of the uniform type bilayer edible sheet. Furthermore, it was confirmed that the stripe type bilayer edible sheet can melt in an oral cavity faster than the uniform type bilayer edible sheet. Therefore, it was found that the bilayer edible sheet produced by streakily applying the mixture of functional ingredients has a variety of superior properties to those of the bilayer edible sheet produced by uniformly applying the mixture of functional ingredients.

The present invention provides an edible sheet, which can be used in a food or medicinal field, exhibiting various stable physical properties even when various functional ingredients are contained therein with base sheet ingredients.

## Claims

1. A bilayer edible sheet obtainable by steps of:
(1) mixing at least one ingredient selected from the group consisting of a starch, a modified starch, a protein, a protein hydrolysate and a gum with water, uniformly applying the mixture on a supporting sheet, and drying the mixture with a hot air to produce a base sheet layer on the supporting sheet;
(2) mixing a surface active agent and at least one functional ingredient selected from the group consisting of a flavoring agent, an acidulant and a pharmaceutically active agent with an organic solvent which does not substantially dissolve the base sheet layer at 30°C to prepare a mixture of functional ingredients;
(3) applying the mixture of functional ingredients prepared in the step (2) on the base sheet layer produced in the step (1), and air-drying the mixture to form a functional ingredient layer on the base sheet layer; and
(4) removing the base sheet layer from the supporting sheet.

2. The edible sheet according to claim 1, wherein the organic solvent in step (2) is selected from the group consisting of acetone, ethyl alcohol, methylene chloride, and esters.

3. The edible sheet according to claim 1, wherein the mixture of functional ingredients is applied using an applicator equipped with a coater or a bar coater, an offset printer, a gravure printer, a screen printer or a sprayer in the step (3).

4. The edible sheet according to claim 1, wherein further at least one ingredient selected from the group consisting of a sweetening agent, a surface active agent, a plasticizer and a coloring agent is mixed with water in the step (1).

5. The edible sheet according to claim 1, wherein further a drying aid is mixed with the organic solvent in the step (2).

6. The edible sheet according to claim 5, wherein the drying aid is a water-insoluble powder having an average diameter of 5-50µ m.

7. The edible sheet according to claim 5, wherein the drying aid is contained in an amount of 0-40% by weight based on a total weight of ingredients contained in the dried edible sheet.

8. The edible sheet according to claim 1, wherein an adhesive is further contained in the mixture of functional ingredients in the step (2).

9. The edible sheet according to claim 8, wherein the adhesive is at least one selected from the group consisting of shellac, cellulose derivative, sucrose fatty acid ester, glycerin fatty acid ester and propylene glycol fatty acid ester.

10. The edible sheet according to claim 8, wherein the adhesive is shellac, and the organic solvent is ethyl alcohol.

11. The edible sheet according to claim 8, wherein the adhesive is contained in an amount of 0-10% by weight based on a total weight of ingredients contained in the dried functional ingredient layer.

12. The edible sheet according to claim 1, wherein a thickness of the dried functional ingredient layer is 5-100µm.

13. The edible sheet according to claim 1, wherein the mixture of functional ingredients is streakily applied on the base sheet layer.

14. The edible sheet according to claim 13, wherein the mixture of functional ingredients is applied in a stripe manner on the base sheet layer.

15. The edible sheet according to claim 1, wherein the mixture of functional ingredients is uniformly applied on the base sheet layer.

16. A process for producing a bilayer edible sheet comprising steps of:
(1) mixing at least one ingredient selected from the group consisting of a starch, a modified starch, a protein, a protein hydrolysate and a gum with water, uniformly applying the mixture on a supporting sheet, and drying the mixture with a hot air to produce a base sheet layer on the supporting sheet;
(2) mixing a surface active agent and at least one functional ingredient selected from the group consisting of a flavoring agent, an acidulant and a pharmaceutically active agent with an organic solvent which does not substantially dissolve the base sheet layer at 30°C to prepare a mixture of functional ingredients;
(3) applying the mixture of functional ingredients prepared in the step (2) on the base sheet layer produced in the step (1), and air-drying the mixture to form a functional ingredient layer on the base sheet layer; and
(4) removing the base sheet layer from the supporting sheet.
